# EUROPÄISCHE PATENTANMELDUNG

(11) **EP 1 210 930 A2**
(43) Veröffentlichungstag der Anmeldung: **05.06.2002**
(21) Anmeldenummer: 01126988.3
(22) Anmeldetag: 14.11.2001
(51) Int. Cl.: A61K 7/06

(54) **Haarbehandlungsmittel und Verfahren zu dessen Herstellung**

(30) Priorität: 23.11.2000 DE 10058037
(71) Anmelder: GOLDWELL GmbH, 64280 Darmstadt (DE)
(72) Erfinder: Heinz, Dieter, 65462 Gustavsburg (DE)

(57) **Zusammenfassung**

Die Erfindung betrifft ein Haarbehandlungsmittel mit vorzüglichen haarkonditionierenden Eigenschaften auf Basis einer homogenen wäßrigen Emulsion bzw. Dispersion, enthaltend
a) 1 bis 25 Gew.-% mindestens einer flüssigen öl- oder fettartigen Substanz;
b) 0,1 bis 10 Gew.-% mindestens eines quaternisierten Polydimethyl- oder Polydiethylamino-C₂-C₃-alkylmethacrylat-Homopolymeren;
c) 0,25 bis 5 Gew.-% mindestens einer langkettigen haarkonditionierenden quaternären Ammoniumverbindung und/oder eines kationischen Polymeren, das sich vom Bestandteil (b) unterscheidet,
jeweils berechnet auf die Gesamtzusammensetzung des Mittels.

Dieses Mittel ist durch einfaches Zusammenrühren bei Raumtemperatur herstellbar und bei Temperaturen bis zu 50°C außergewöhnlich viskositätsstabil.

## Beschreibung

Die vorliegende Erfindung betrifft ein Haarpflegemittel in Form einer wäßrigen Emulsion bzw. Dispersion mit verbesserten haarkonditionierenden und galenischen Eigenschaften.

Es ist seit Jahrzehnten bekannt, Haarkonditioniermittel der verschiedensten Art zu benutzen. Sie enthalten in der Regel kationische Wirkstoffe, vor allem quaternäre langkettige Ammoniumverbindungen, und/oder Polymere mit quaternären Stickstoffatomen und werden insbesondere als Lösungen, Öl-in-Wasser-Emulsionen, Gele oder Lotionen eingesetzt, die auch als Aerosole oder Pumpsprays zum Einsatz gelangen können.

Entsprechende Zusammensetzungen, die entweder nach der Applikation im Haar verbleiben oder aber auch ausgewaschen werden können, sind beispielsweise in der Monographie von K. Schrader, Grundlagen und Rezepturen der Kosmetika, 2. Aufl., (Hüthig Buch Verlag, 1989) auf den Seiten 722 bis 781 unter dem Stichwort "Haarnachbehandlungsmittel" beschrieben.

Trotz ihrer Vielfalt sind diese Produkte hinsichtlich ihrer anwendungstechnischen Eigenschaften noch immer verbesserungsfähig.

Es wurde nunmehr festgestellt, und das ist Gegenstand der vorliegenden Erfindung, daß ein optimales konditionierendes Haarnachbehandlungsmittel aus einer wäßrigen Emulsion bzw. Dispersion besteht, die
a) 1 bis 25 Gew.-% mindestens einer öl- oder fettartigen Substanz;
b) 0,1 bis 10 Gew.-% mindestens eines quaternisierten Polydimethyl- oder Polydiethylamino-C₂-C₃-alkylmethacrylat-Homopolymeren;
c) 0,25 bis 5 Gew.-% mindestens einer langkettigen haarkonditionierenden Ammoniumverbindung und/oder eines kationischen Polymeren, das sich vom Bestandteil (b) unterscheidet,
   enthält.

Dieses Produkt verleiht dem Haar nicht nur Glanz, Fülle und Volumen, einen vollen Griff, Spannkraft und verbesserte Naß- und Trockenkämmbarkeit, sondern weist auch keinerlei sonst bei solchen Produkten häufig auftretende Klebrigkeit oder Hygroskopizität auf dem Haar auf.

Darüber hinaus ist diese Emulsion bzw. Dispersion außergewöhnlich homogen und bis 50°C viskositätsstabil; sie kann auch bei Normaltemperatur durch einfaches Zusammenrühren der Bestandteile ohne den ansonsten obligatorischen Zusatz eines W/O- oder O/W-Emulgators hergestellt werden.

Der bevorzugte Anteil an quaternisiertem Polydimethyl- oder Polydiethylamino-C₂-C₃-alkylmethacrylat liegt bei 0,25 bis 5, insbesondere 0,5 bis 2,5 Gew.-%, berechnet auf die Gesamtzusammensetzung.
Derartige Polydimethyl- bzw. Polydiethylaminoethyl- bzw. -aminopropylmethacrylate werden durch Polymerisation der entsprechenden Monomeren gewonnen und sind auch auf dem Markt erhältlich, z.B. als 50%-ige Dispersion in organischen Lösungsmitteln, z.B. flüssigen Fettsäureestern und/oder Fettalkoholen.
Besonders bevorzugt ist die Verwendung eines mit Methylchlorid oder Dimethyl-oder Diethylsulfat quaternisierten Dimethylaminoethylmethacrylat-Homopolymerisats als 40- bis 60%-ige Dispersion in einem flüssigen 1,2-Propandioldi-C₆-C₁₂-fettsäureester wie 1,2-Propandioldicaprylat/dicaprat.

Die Verwendung dieser Verbindung in Haarpflegemitteln ist aus der WO 99/26592 A1 grundsätzlich bekannt.
Dort findet sich jedoch keinerlei Hinweis auf die speziellen erfindungsgemäßen Zusammensetzungen und deren besondere Wirkungen.

Diese Zusammensetzungen sind, wie bereits ausgeführt, bis zu Temperaturen von 50°C außergewöhnlich viskositätsstabil.

Der Viskositätsbereich des Mittels liegt dabei bei etwa 10000 bis 200 000 mPa.s bei 20°C (Brookfield RVT; Spindel Nr. 5 oder 6), vorzugsweise etwa 15 000 bis 150 000 mPa.s, insbesondere etwa 20 000 bis 100 000 mPa.s, gemessen jeweils bei 20°C.

Der Anteil der Ölphase, d.h. der flüssigen öl- oder fettartigen Substanz, in den erfindungsgemäßen Zusammensetzungen beträgt 1 bis 25, vorzugsweise etwa 2,5 bis 15, insbesondere etwa 3 bis 10 Gew.-% der Gesamtzusammensetzung.

Als flüssige Fette und Öle werden solche mit einem Schmelzpunkt von maximal etwa 30°C, vorzugsweise etwa 25°C, angesehen.
Geeignete Fette und Öle sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, Purcellinöl (Cetearyloctanoat) oder auch Oliven- bzw. Sojaöl, sowie sonstige Mono-, Di- und Triglyceride, Lanolin und dessen Derivate, ebenso Mineralöle wie Paraffinöl und Vaseline.
Synthetische Öle und Wachse sind beispielsweise Silikonöle, Polyethylenglykole, etc.
Weitere geeignete hydrophobe Komponenten sind insbesondere Fettsäureester wie Ethyl-, n-Propyl- und Isopropylmyristat, -palmitat, -stearat, -laurat, -oleat und -isostearat, Decyloleat, Oleyloleat, Isocetylstearat, Hexyllaurat, Decylstearat, Dibutyladipat, Oleyladipat, Dioctyladipat, Tristearylcitrat, Myristylmyristat, Cetylpropionat, Oleylerucat, Polyethylenglykol- und Polyglycerylfettsäureester wie PEG-7-glycerylcocoat, etc.

Weitere geeignete Substanzen sind Fettalkohole wie Oleylalkohol, Decyl- und Laurylalkohol und natürliche Gemische derselben wie Cocosfettalkohol, die auch ethoxyliert sein können.

Wie bereits ausgeführt, ist die Anwesenheit von Emulgatoren zur Herstellung der erfindungsgemäßen Emulsion bzw. Dispersion nicht unbedingt erforderlich.
Falls erwünscht, können jedoch oberflächenaktive Stoffe, insbesondere nichtionische, amphotere oder zwitterionische, zugesetzt werden.
Solche nichtionischen Tenside sind beispielsweise Fettalkoholpolyglykolether.

Besonders geeignete C₁₀-C₂₂-Fettalkoholether sind die unter den Trivialnamen "Laureth", "Myristeth", "Oleth", "Ceteth", "Deceth", "Steareth", "Trideceth" und "Ceteareth" nach der CTFA-Nomenklatur mit Anfügung der Zahl der Ethylenoxid-Moleküle bezeichneten Alkylpolyglykolether, z.B. "Laureth-16" und "Trideceth-6".
Der durchschnittliche Ethoxylierungsgrad liegt dabei zwischen etwa 2,5 und etwa 25, vorzugsweise etwa 5 und etwa 20.

Geeignete weitere nichtionische Tenside sind Verbindungen aus der Klasse der Alkylpolyglucoside mit der allgemeinen Formel

R-0-(CH₂CH₂0)ₙ-Zₓ,

worin R eine Alkylgruppe mit 8 bis 20, vorzugsweise 10 bis 14 Kohlenstoffatomen, Zₓ einen Saccharidrest mit 5 bis 6 Kohlenstoffatomen, n, eine Zahl von 0 bis 10, und x eine Zahl zwischen 1 und 5, vorzugsweise 1,1 bis 2,5 bedeuten.
Andere zusätzlich mitverwendbare nichtionische Tenside sind z.B. die verschiedenen Sorbitanester wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics^{R}" im Verkehr sind.

Weitere einsetzbare nichtionische Tenside sind Aminoxide.
Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder ethylaminoxide, C₁₂-C₁₈-Alkyldi(hydroxyethyl)- oder -(hydroxypropyl)aminoxide, oder auch Aminoxide mit Ethylenoxid- und/oder Propylenoxidgruppen in der Alkylkette.
Geeignete Aminoxide sind beispielsweise unter den Bezeichnungen "Ammonyx® ", "Aromox® ", oder "Genaminox® " im Handel.

Geeignete amphotere bzw. zwitterionische Tenside sind insbesondere die verschiedenen bekannten Betaine wie Fettsäureamidoalkylbetaine und Sulfobetaine, beispielsweise Laurylhydroxysulfobetain; auch langkettige Alkylaminosäuren wie Cocoaminoacetat, Cocoaminopropionat und Natriumcocoamphopropionat und -acetat haben sich als geeignet erwiesen.
In einzelnen können Betaine der Struktur 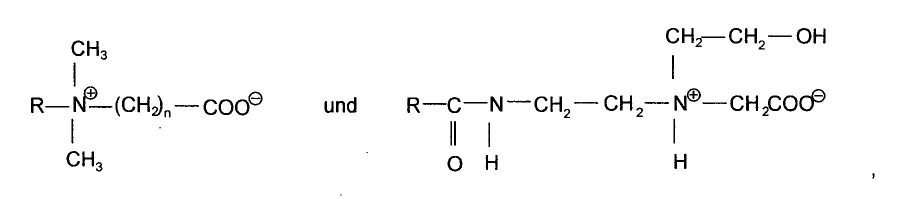 wobei R eine C₈-C₁₈-Alkylgruppe und n 1 bis 3 bedeuten,

Sulfobetaine der Struktur 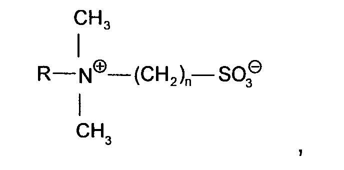 wobei R eine C₈-C₁₈-Alkylgruppe und n 1 bis 3 bedeuten,
und Amidoalkylbetaine der Struktur 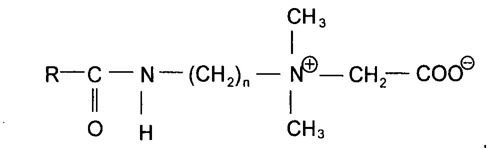 wobei R eine C₈-C₁₈-Alkylgruppe und n 1 bis 3 bedeuten, verwendet werden.

Bevorzugt sind Fettsäureamidoalkylbetaine, insbesondere Cocoamidopropylbetain, und Cocoamphoacetat und -propionat, insbesondere deren Natriumsalze.
Besonders bevorzugt sind Gemische aus Cocoamidopropylbetain und Cocoamphoacetat, insbesondere im Gewichtsverhältnis 3:1 bis 1:3, vor allem 2:1 bis 1:1.

Der dritte obligatorische Bestandteil des erfindungsgemäßen Haarbehandlungs-mittels ist mindestens eine haarkonditionierende langkettige quaternäre Ammonium-verbindung und/oder ein kationisches Polymer, das sich vom ebenfalls obligatorisch anwesenden kationischen Polymeren, dem quaternisierten Homopolymerisat eines Dimethyl- oder Diethylamino-C₂-C₃-alkylmethacrylats, natürlich unterscheidet.

Geeignete haarkonditionierende langkettige quaternäre Ammoniumverbindungen, die allein oder im Gemisch miteinander eingesetzt werden können, sind Cetyltrimethylammoniumchlorid, Dimethyldiethylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxyethyl)alkylammoniumphosphat, Cetylpyridiniumchlorid, etc.
Gut geeignet sind auch die in der EP-A 472 107 geoffenbarten quaternären Ammoniumsalze.

Weitere geeignete langkettige Ammoniumverbindungen sind Esterquats der allgemeinen Formel (I) 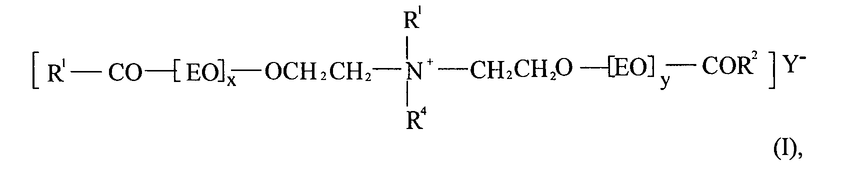 in der R¹ und R² für eine gegebenenfalls hydroxysubstituierte C₈-C₂₂-Alkyl-oder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y⁻ für ein Anion stehen.

Eine besonders bevorzugte Verbindung der Formel I ist im Rahmen der Erfindung eine solche, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[EO]_{z}-H bedeuten.

Das Anion Y⁻ ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.

Diese Verbindungen sind an sich bekannt und beispielsweise unter den Handelsnamen "Schercoquat^{R}", "Dehyquart^{R}F30" und "Tetranyl^{R}" im Handel.

Der Einsatz dieser "Esterquats" in Haarpflegemitteln ist an sich bekannt und beispielsweise in der WO-A 93/10748, der WO-A 92/06899 und der WO-A 94/16677 beschrieben.

Geeignet sind auch Amidoquats der allgemeinen Formel (II) 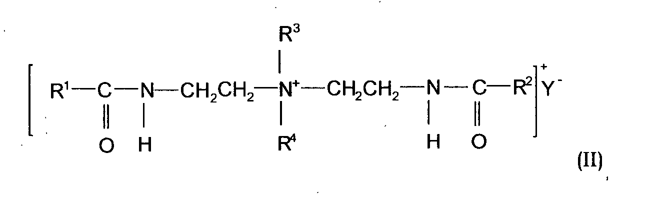 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe-CH₂-CH₂-O-[EO]ₓ-H, sowie x für 0 bis 5, und Y⁻ für ein Anion stehen.

Es können selbstverständlich auch Gemische aus den verschiedenen quaternären Ammoniumverbindungen verwendet werden.

Anstelle oder zusätzlich zu den haarkonditionierenden langkettigen Ammoniumverbindungen können auch haarkonditionierende kationische Polymere eingesetzt werden.

Bevorzugte konditionierende kationische Polymere sind die altbekannten quaternären Cellulosederivate des Typs "Polymer JR" sowie quaternisierte Homo- und Copolymere des Dimethyldiallylammoniumchlorids, wie sie unter dem Handelsnamen "Merquat® " im Handel sind, quaternäre Vinylpyrrolidon-Copolymere, insbesondere mit Dialkylaminoalkyl(meth)acrylaten, wie sie unter dem Namen "Gafquat® " bekannt sind, Copolymerisate aus Vinylpyrrolidon und Vinylimidazoliniummethochlorid, die unter dem Handelsnamen "Luviquat® " angeboten werden, Polyamino-Polyamid-Derivate, beispielsweise Copolymere von Adipinsäure-Dimethylaminohydroxypropyldiethylentriamin, wie sie unter dem Namen "Cartaretine® F" vertrieben werden, sowie auch bisquaternäre langkettige Ammoniumverbindungen der in der US-PS 4 157 388 beschriebenen Harnstoff-Struktur, die unter dem Handelsnamen "Mirapol® A 15" erhältlich sind.
Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.
Zu den kationischen Polymeren zählen auch die in der EP-A 524 612 und der EP-A 640 643 beschriebenen Quaternisierungsprodukte aus Pfropfpolymerisaten von Organopolysiloxanen und Polyethyloxazolinen.

Der Anteil dieser haarkonditionierenden Substanzen beträgt etwa 0,1 bis 10, vorzugsweise 0,25 bis 7,5, insbesondere 0,5 bis 5 Gew.-% der Gesamtzusammensetzung.

Die erfindungsgemäßen Zusammensetzungen können auch weitere Polymere, insbesondere nichtionische, amphotere und/oder zwitterionische, enthalten.
Diese Polymeren sind dem Fachmann bekannt und auf dem Markt erhältlich.

Das erfindungsgemäße Haarbehandlungsmittel kann als weiteren Bestandteil noch mindestens eine Verbindung, ausgewählt aus der Gruppe 1-Methoxypropanol(-2), 1-Ethoxypropanol(-2), Diethylenglykolmonomethyl- oder -ethylether, Dipropylenglykolmonomethyl- oder -ethylether, Benzylalkohol, Benzyloxyethanol, Phenylethylalkohol, Phenoxyethanol und/oder Zimtalkohol, vorzugsweise in einer Menge von 0,5 bis 25, insbesondere 1 bis 20, vor allem 2,5 bis 15 Gew.-%, berechnet auf die Gesamtzusammensetzung des Mittels, enthalten.
Bevorzugte Verbindungen aus dieser Gruppe sind Ethoxydiglykol und Benzyloxyethanol.

Dabei gilt grundsätzlich, daß wasserlösliche Ingredienzien in der Wasserphase und wasserunlösliche Ingredientien in der Öl- bzw. Fettphase der erfindungsgemäßen Dispersion bzw. Emulsion enthalten sind, wobei natürlich auch Grenzfälle auftreten können.

Ein weiterer möglicher Bestandteil in diesen Mitteln ist Harnstoff, vorzugsweise in einer Menge von etwa 1 bis 10, insbesondere etwa 2,5 bis 7,5 Gew.-% des erfindungsgemäßen Mittels.

Die erfindungsgemäßen Mittel können neben den essentiellen zusätzlich auch noch weitere konditionierende wasserlösliche Eiweißhydrolysate und Polypeptide, z.B. Keratinhydrolysate, Kollagenhydrolysate vom Typ "Nutrilan® " oder Elastinhydrolysate sowie insbesondere auch pflanzliche, gegebenenfalls kationisierte Eiweißhydrolysate, z.B. "Gluadin^{R}", enthalten.

Des weiteren können alle in solchen Zusammensetzungen üblichen Bestandteile anwesend sein.
Als solche seien beispielhaft Komplexbildner, Farbstoffe, Konservierungsmittel, pH-Regler, Viskositätsregler wie anorganische Salze, Duftstoffe, Perlglanzmittel, Verdickungsmittel, Feuchthaltemittel, Cholesterine wie Lecithin und dessen Derivate etc. genannt.
Eine Auflistung solcher Zusatzstoffe findet sich ebenfalls bei Schrader, l.c., auf S.695 bis 722.
Schließlich können auch noch bekannte Polysiloxane als konditionierende Mittel in den erfindungsgemäßen Haarpflegeemulsionen und -dispersionen, vorzugsweise in der Öl- bzw. Fettphase, mitverwendet werden. Deren bevorzugter Anteil liegt dabei etwa zwischen 0,5 und etwa 5, insbesondere 1 bis 3 Gew.-% der Gesamtzusammensetzung.

Geeignet sind sowohl leichtflüchtige als auch schwerflüchtige cyclische oder lineare Polysiloxane, beispielsweise die unter den Trivialnamen "Dimethicone" bzw. "Phenyldimethicone" sowie "Cyclomethicone" bekannten Silikonöle, sowie beispielsweise auch die in der EP-A 398 177 beschriebenen Silikonderivate, die dort in Kombination mit Alkylpolyglucosiden in flüssigen Detergens-Zusammensetzungen eingesetzt werden.

Ein weiterer möglicher Bestandteil in den erfindungsgemäßen Mitteln sind Pflanzenextrakte in einer Menge von etwa 0,01 bis etwa 10, vorzugsweise 0,1 bis 7,5, insbesondere 0,5 bis 5 Gew.-%, berechnet als Trockenrückstand desselben auf die Gesamtmenge des Mittels, die vorzugsweise in der wäßrigen Phase der erfindungsgemäßen Emulsion bzw. Dispersion vorliegen.

Geeignete, an sich bekannte wäßrige (z.B. Wasserdampf-destillierte), alkoholische oder wäßrig-alkoholische Pflanzenextrakte sind insbesondere Extrakte von Blättern, Früchten, Blüten, Wurzeln, Rinden oder Stämmen von Aloe, Ananas, Artischocken, Arnika, Avocado, Baldrian, Bambus, Bilsenkraut, Birke, Brennesseln, Echinacea, Efeu, Engelwurz, Enzian, Farnen, Fichtennadeln, Gänsefingerkraut, Ginseng, Ginster, Hafer, Hagebutten, Hamamelis, Heublumen, Holunder, Hopfen, Huflattich, Johannisbeeren, Kamillen, Karotten, Kastanien, Klee, Klettenwurzeln, Kokosnuß, Korn, Lindenblüten, Maiglöckchen, Meeralgen, Melisse, Mistel, Passionsblumen, Ratanhia, Ringelblumen, Rosmarin, Roßkastanien, Rotdorn, Salbei, Schachtelhalm, Scharfgarbe, Schlüsselblumen, Taubnesseln, Thymian, Walnüssen, Weinblättern, Weißdorn, etc.

Geeignete Handelsprodukte sind beispielsweise die verschiedenen "Extrapone^{R}", "Herbasol^{R}", "Sedaplant^{R}" und "Hexaplant^{R}". Extrakte und deren Herstellung sind auch in "Hagers Handbuch der pharmazeutischen Praxis", 4. Aufl., beschrieben.
Ein weiterer wirkungssteigernder Bestandteil in den erfindungsgemäßen Zusammensetzungen sind Mono- und Oligosaccharide in einer Menge zwischen etwa 0,05 und 5, insbesondere 0,1 bis 4, vorzugsweise 0,25 bis 2,5 Gew.-% der Gesamtzusammensetzung.
Bevorzugte Substanzen sind dabei Glucose, Saccharose, Fructose, Maltose, Lactose, Cellobiose und insbesondere Fucose.

Die Herstellung der erfindungsgemäßen wäßrigen Dispersionen bzw. Emulsionen erfolgt, wie bereits erwähnt, durch einfaches Zusammenrühren des Öls bzw. der fettartigen Substanz mit den restlichen Bestandteilen der Zusammensetzung ohne zusätzliche Erwärmung und anschließenden Zusatz des quaternisierten Dimethyl-bzw. Diethylaminoethyl- bzw. -propylmethacrylat-Homopolymeren, wobei es ebenfalls keiner zusätzlichen Erhitzung bedarf.
Es wird ein viskositätsstabiles homogenes Produkt enthalten.

Im folgenden werden einige Beispiele für erfindungsgemäße Zusammensetzungen gegeben.

### Zusammensetzungen Nr.

| | **1** | **2** | **3** | **4** | **5** |
|---|---|---|---|---|---|
| PCL-Liquid | 4,0 | 5,0 | 6,0 | 4,0 | 3,5 |
| Laurylpyrrolidon | 0,2 | 0,2 | 0,2 | 0,2 | 0,2 |
| Octylmethoxylcinnamat | 0,5 | 0,5 | 0,5 | 0,5 | 0,5 |
| Parfumöl | 0,4 | 0,4 | 0,4 | 0,4 | 0,4 |
| Weizenkleielipide, öllöslich | 0,4 | 0,5 | 0,6 | 0,4 | 0,4 |
| 1,2-Propandioldicaprylat/dicaprat | 1,0 | 1,5 | 0,8 | 1,0 | 1,5 |
| Trideceth-6 | 0,2 | 0,3 | - | 0,2 | - |
| Laureth-12 | - | - | 0,5 | - | 0,3 |
| Lecithine | 0,3 | 0,3 | 0,3 | 0,3 | 0,3 |
| 1,2-Propandiol | - | - | 0,4 | - | - |
| Polyquaternium-37 | 1,0 | 1,2 | 0,8 | 1,0 | 1,5 |
| Dicocoylethylhydroxyethylmoniummethosulfat | - | - | 1,6 | - | - |
| Cetrimoniumchlorid | - | 1,0 | - | - | - |
| Lauryltrimethylammoniumchlorid | 1,0 | - | - | - | - |
| Polyquaternium-7 | - | - | - | 1,0 | |
| Polyquaternium-6 | - | - | - | - | 0,5 |
| Wasser ad | 100,0 | 100,0 | 100,0 | 100,0 | 100,0 Gew.-% |
| Brookfield Viskosität bei 20°C (Spindel Nr.5) | 26000 | 29000 | 15000 | 16000 | 80000 mPa·s |

Die Bestandteile wurden unter Rühren mit Wasser vermischt und anschließend das Polyquaternium-37, dispergiert in 1,2-Propandioldicaprylat/dicaprat und Trideceth-6 bzw. Laureth-12, bei Raumtemperatur unter Rühren zugesetzt.

Es wurde eine feine, weiße, sehr homogene Dispersion, deren Viskosität sich bei sechsmonatiger Lagerung bei 40°C nicht veränderte, erhalten.

Die Zusammensetzungen eignen sich vorzüglich als Haarkuren, die entweder im Haar verbleiben oder auch nach der Einwirkung ausgespült werden können.

## Patentansprüche

1. Haarbehandlungsmittel auf Basis einer wäßrigen Emulsion bzw. Dispersion, enthaltend
a) 1 bis 25 Gew.-% mindestens einer flüssigen öl- oder fettartigen Substanz;
b) 0,1 bis 10 Gew.-% mindestens eines quaternisierten Polydimethyl- oder Polydiethylamino-C₂-C₃-alkylmethacrylat-Homopolymeren;
c) 0,25 bis 5 Gew.-% mindestens einer langkettigen haarkonditionierenden quaternären Ammoniumverbindung und/oder eines kationischen Polymeren, das sich vom Bestandteil (b) unterscheidet,
jeweils berechnet auf die Gesamtzusammensetzung des Mittels.

2. Haarbehandlungsmittel nach Anspruch 1, enthaltend als Bestandteil (b) 0,25 bis 5 Gew.-% quaternisiertes Polydimethylaminoethylmethacrylat.

3. Haarbehandlungsmittel nach Anspruch 1 und/oder Anspruch 2, enthaltend den Bestandteil b) als 40 bis 60-prozentige Dispersion in einem flüssigen 1,2-Propandioldi-C₆-C₁₂-fettsäureester.

4. Haarbehandlungsmittel nach einem oder mehreren der Ansprüche 1 bis 3, das eine Brookfield-Viskosität zwischen 10 000 und 200 000 mPa.s bei 20°C aufweist.

5. Haarbehandlungsmittel nach einem oder mehreren der Ansprüche 1 bis 4, enthaltend 0,05 bis 5 Gew.-%, berechnet auf die Gesamtzusammensetzung, mindestens eines Mono- und/oder Oligosaccharids.

6. Verfahren zur Herstellung eines Haarbehandlungsmittels nach einem oder mehreren der Ansprüche 1 bis 5, wobei unter Rühren die flüssigen Öl- und fettartigen Substanzen mit den restlichen Bestandteilen der Zusammensetzung ohne zusätzliche Erwärmung vermischt und anschließend der Bestandteil b) zugesetzt und die Mischung zu einer homogenen Emulsion bzw. Dispersion gerührt wird.
